# FASCICULE DE BREVET EUROPEEN

(11) **EP 1 370 145 B1**
(45) Date de publication et mention de la délivrance du brevet: **05.12.2007**
(21) Numéro de dépôt: 02720052.6
(22) Date de dépôt: 12.03.2002
(51) Int. Cl.: A23B 9/02, A23B 7/005, A61L 2/06, A23L 3/18, A23L 3/01

(54) **PROCEDE DE DECONTAMINATION MICROBIOLOGIQUE DE PRODUITS PULVERULENTS**
VERFAHREN ZUM MIKROBIOLOGISCHEN DEKONTAMINIEREN VON PRODUKTEN IN PULVERFORM
METHOD FOR MICROBIOLOGICAL DECONTAMINATION OF POWDERY PRODUCTS

(30) Priorité: 13.03.2001 FR 0103366
(43) Date de publication de la demande: 17.12.2003
(73) Titulaire: UNIVERSITE DE BOURGOGNE, F-21000 Dijon (FR)
(72) Inventeur: GERVAIS, Patrick, F-21800 Quetigny (FR); MARECHAL, Pierre-André, F-21000 Dijon (FR); PERRIER-CORNET, Jean-Marie, F-21160 Perrigny (FR); FERRET, Eric, F-21000 Dijon (FR); LAROCHE, Céline, F-21000 Dijon (FR)
(74) Mandataire: Honoré, Anne-Claire
(86) Numéro de dépôt international: PCT/FR2002/000873
(87) Numéro de publication internationale: WO 2002/071853

(56) Documents cités:
- EP-A- 0 534 051
- EP-A- 0 556 101
- EP-A- 0 722 669
- WO-A-00/74493
- WO-A-96/20606
- WO-A-99/03355
- DE-A- 4 424 430
- US-A- 3 812 595
- US-A- 4 844 933
- US-A- 5 066 506
- US-A- 5 082 679
- US-A- 5 193 350

## Description

La présente invention concerne un procédé de décontamination microbiologique de produits pulvérulents, de graines ou analogues, afin de détruire en tout ou partie les levures, les moisissures, les bactéries ou similaires, initialement présentes sur le produit pulvérulent tout en préservant ses qualités organoleptiques ainsi qu'un dispositif mettant en oeuvre le procédé.

Dans le domaine de l'alimentation, on connaît déjà un procédé de décontamination microbiologique, dît "procédé par ionisation" particulièrement adapté à la décontamination des produits pulvérulents tels que des graines, des poudres ou similaires. Ce procédé consiste à bombarder le produit de radiations ionisantes créées par accélération d'électrons, par isotopes radioactifs ou par une source de rayons X qui détruisent les microbes, les bactéries, les insectes, etc ...

Même si l'aspect visuel et les qualités organoleptiques des produits traités restent inchangés après une décontamination par ionisation et dans la mesure où, la législation en vigueur dans de nombreux pays oblige les fabriquants de denrées alimentaires à signaler sur leurs emballages l'utilisation d'un tel procédé de décontamination, les consommateurs, effrayés par l'utilisation d'éléments radioactifs dans ce procédé, rejettent les produits décontaminés ainsi traités.

On connaît, par ailleurs, des procédés substitutifs de décontamination utilisant de l'oxyde d'éthylène, de l'oxyde de propylène, du dioxyde de souffre, de l'éthanol, du bromure de méthyle ou d'hypochlorite. Ces procédés, outre leurs durées de traitement et leurs coûts excessifs qui grèvent considérablement les coûts de production, présentent l'inconvénient de dénaturer les qualités organoleptiques ou l'apparence des produits, de polluer l'environnement et d'accroître le risque de cancer des personnes consommant des produits traités suivant ces procédés, de sorte que la plupart de ces procédés sont interdits, notamment en Europe.

Enfin, on connaît également des procédés de décontamination de produits alimentaires qui consistent à chauffer lesdits produits puis à les refroidir ; c'est le cas, par exemple, du brevet allemand DE 44.24430 décrivant un procédé pour la décontamination de produits végétaux séchés. Le procédé consiste à placer des produits dans un flux de gaz à une température comprise entre 80 et 170°C pendant 30 secondes à 3 minutes puis à refroidir lesdits produits en les immergeant dans du CO₂ liquide placé dans une enceinte sous pression contenant une vis sans fin, la température du CO₂ étant d'environ -50°C. Ce type de procédé ne permet de réduire la flore totale, c'est-à-dire les levures et les moisissures des produits alimentaires que très partiellement et en tout les cas de manière tout à fait insuffisante, de sorte que ces produits deviennent rapidement impropres à la consommation lorsqu'ils sont stockés à l'air libre.

L'un des buts de l'invention est donc de remédier à ces inconvénients en proposant un procédé de décontamination microbiologique de produits pulvérulents détruisant tout ou partie des levures, des moisissures, des bactéries ou similaires, dans un temps très court tout en préservant les qualités organoleptiques du produit.

A cet effet, le procédé de décontamination microbiologique de produits pulvérulents, de graines ou analogues est remarquable en ce qu'il consiste au moins à soumettre le produit pulvérulent à un premier traitement thermique consistant à appliquer un flux de gaz chaud sur le produit pulvérulent à une température comprise entre 200°C et 600°C pendant une durée inférieure 30 secondes puis, immédiatement après, à soumettre ledit produit à un second traitement thermique consistant à appliquer un flux de gaz froid sur le produit pulvérulent à une température inférieure à -80°C pendant une durée inférieure à 30 secondes, afin de détruire en tout ou partie les levures, les moisissures, les bactéries ou similaires, initialement présentes sur le produit pulvérulent tout en conservant ses qualités organoleptiques.

On comprend bien que le premier traitement thermique chaud permet de détruire les levures, moisissures, les bactéries ou analogues tout en évitant de brûler le produit pulvérulent et que le second traitement froid permet d'arrêter la progression de la chaleur dans le produit pulvérulent évitant ainsi de le calciner.

Selon une autre variante d'exécution du procédé de décontamination microbiologique, le premier et le second traitement thermique consistent à faire transiter le produit pulvérulent dans une enceinte contenant un gaz chaud et respectivement froid.

La température du gaz, lors des premier et second traitements thermiques est avantageusement maintenue globalement constante. De plus, la température du gaz du premier traitement thermique est de préférence comprise entre 300° et 400°, et la température du gaz du second traitement thermique est comprise entre -200° et -80°C.

Selon une dernière variante d'exécution du procédé, ledit procédé consiste à transporter le produit pulvérulent dans un flux de gaz froid et à appliquer sur au moins une partie de son trajet un rayonnement apte à chauffer ledit produit pulvérulent. Il peut s'agir d'un rayonnement de micro-ondes, d'infrarouges, d'ultraviolets ou analogues.

Un autre objet de l'invention concerne un dispositif de décontamination microbiologique de produits pulvérulents, de graines ou analogues mettant en oeuvre le procédé.

Le dispositif est constitué d'un premier tuyau de transport pneumatique relié à une trémie via une écluse qui consiste dans un conduit muni de deux vannes, ledit premier tuyau de transport pneumatique comprenant à une première extrémité des moyens de ventilation et des moyens de chauffage et à son extrémité opposée un premier cyclone comprenant une première sortie de fluide de transport et une seconde sortie des produits pulvérulents, ladite seconde sortie débouchant sur un conduit, ledit conduit étant relié à un second tuyau de transport pneumatique, comprenant à une première extrémité des moyens de ventilation et des moyens de refroidissement à proximité dudit conduit et à son extrémité opposée un second cyclone comprenant une première sortie du fluide de transport et une seconde sortie des produits pulvérulents, ladite seconde sortie débouchant sur des moyens de récupération.

D'autres avantages et caractéristiques ressortiront mieux encore de la description qui va suivre de plusieurs variantes d'exécution, données à titre d'exemples non limitatifs, du procédé de décontamination microbiologique de produits pulvérulents et du dispositif le mettant en oeuvre conformément à l'invention en référence aux dessins annexés sur lesquels:
- la figure 1 est une représentation schématique d'un dispositif permettant de mettre en oeuvre le procédé conforme à l'invention,
- la figure 2 est une représentation schématique d'une variante d'exécution d'un dispositif permettant de mettre en oeuvre le procédé selon l'invention,
- la figure 3 est une représentation schématique d'un dispositif selon l'invention mettant en oeuvre le procédé conforme à l'invention,
- la figure 4 est une représentation schématique d'une dernière variante d'exécution d'un dispositif permettant de mettre en oeuvre le procédé conforme à l'invention.

On décrira, à titre d'exemple non limitatif différents dispositifs permettant de mettre en oeuvre le procédé de décontamination microbiologique conforme à l'invention pour le traitement de grains de poivre entier.

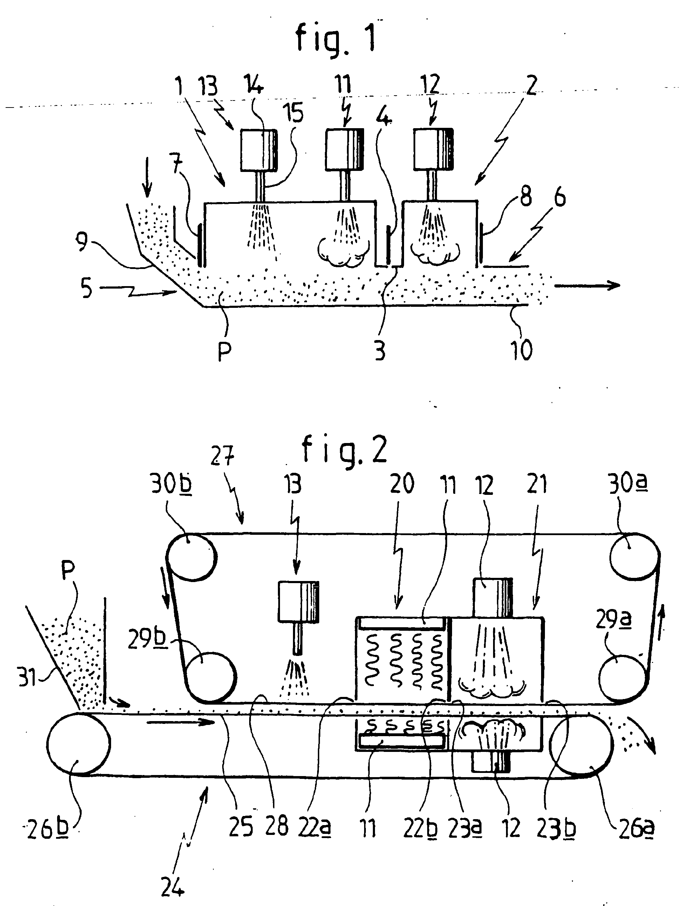

Selon une première variante d'exécution du dispositif, représenté sur la figure 1, celui-ci est constitué de deux enceintes 1 et 2 de préférence isothermes reliées entre elles par un conduit 3 muni d'une vanne 4. Lesdites enceintes 1 et 2 comprennent respectivement une entrée 5 et une sortie 6 munies de moyens de fermeture 7 et respectivement 8 tels qu'une vanne par exemple, pour permettre l'introduction et le retrait de grains de poivre P dans lesdites enceintes 1 et 2. Les grains de poivre P sont avantageusement transportés dans la première enceinte 1 par un premier tuyau de transport pneumatique 9 connecté à l'entrée 5 et sont retirés de la seconde enceinte 2 par un second tuyau de transport pneumatique 10 relié à la sortie 6. La première enceinte 1 comprend, par ailleurs, des moyens de chauffage 11 du gaz contenu à l'intérieur de ladite enceinte 1, en l'occurrence de l'air, lesdits moyens 11 étant par exemple constitués de moyens de ventilation et d'une thermo-résistance afin de projeter dans l'enceinte 1 de l'air chauffé par la résistance thermique à une température comprise entre 200°C et 600°C. De plus, la seconde enceinte 2 comprend des moyens de refroidissement 12 constitués, par exemple, d'une bouteille de CO2 comprimé, d'un plongeur et d'un détendeur rejetant dans l'enceinte 1 le CO2 détendu à une température voisine de -78°C.

Il va de soi que la température du CO2 rejeté dans l'enceinte 1 peut être contrôlée en faisant varier l'arrivée d'air lors de la détente du CO2.

Par ailleurs, il est bien évident que le dispositif peut consister dans une unique enceinte isotherme comprenant les moyens de chauffage 11 et de refroidissement 12 sans pour autant sortir du cadre de l'invention.

Accessoirement, le dispositif comprend des moyens d'humidification 13 constitués d'un réservoir d'eau 14 et d'une buse 15 apte à permettre la pulvérisation de l'eau sur les grains de poivre P contenues dans l'enceinte 1 afin de palier si nécessaire à leur déshydratation, comme on le verra plus loin.

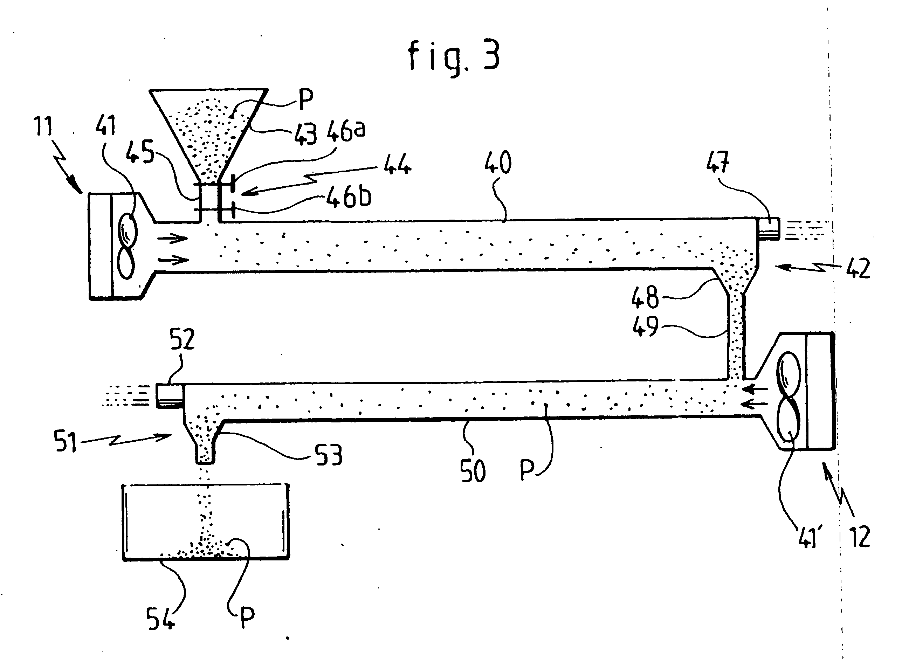

Selon une variante d'exécution du dispositif de décontamination, représenté sur la figure 2, le dispositif est constitué de deux enceintes 20,21 contigues et de préférence isothermes comprenant respectivement une entrée 22a,23a et une sortie 22b,23b, la sortie 22b de l'enceinte 20 correspondant à l'entrée 23a de l'enceinte 21. Le dispositif comprend, de la même manière que précédemment, des moyens de chauffage 11 aptes à réchauffer l'air contenu dans l'enceinte 20 et des moyens de refroidissement 12 aptes à refroidir l'air contenu dans l'enceinte 21. Par ailleurs, le dispositif comprend des moyens de convoyage consistant d'une part dans un premier convoyeur inférieur sans fin 24 comprenant un tapis transporteur 25 passant sur un rouleau aval menant 26a et un rouleau amont 26b et, d'autre part, dans un second convoyeur supérieur sans fin 27 comprenant un tapis transporteur 28 passant sur deux rouleaux avals menants 29a, 30a et deux rouleaux amonts 29b, 30b. les grains de poivre P initialement contenus dans une trémie 31 sont positionnés sur le tapis 25 du convoyeur inférieur 24 en amont du convoyeur supérieur 27. De plus, le tapis supérieur 28 s'étend parallèlement au tapis inférieur 25 de sorte que les grains de poivre P sont maintenus en position entre les tapis 25,28 depuis l'entrée 22a jusqu'à la sortie 23b des enceintes 20,21. Les tapis inférieur et supérieur 25,28 consistent avantageusement dans du grillage dont la maille est inférieure au diamètre des grains de poivre P afin de permettre la diffusion de l'air chaud et de l'air froid jusque dans la partie centrale de l'espace délimité entre lesdits tapis inférieur 25 et supérieur 28.

Accessoirement, le dispositif comprend des moyens d'humidification 13 des grains de poivre P positionnés en amont des enceintes 20,21.

Selon une variante d'exécution du dispositif conforme à l'invention en référence à la figure 3, le dispositif est constitué d'un premier tuyau de transport pneumatique 40 dont les parois sont avantageusement calorifugées comprenant à une première extrémité des moyens de ventilation 41 et des moyens de chauffage 11 et à son extrémité opposée un cyclone 42 permettant de séparer les grains de poivre P du fluide de transport chaud, la température du fluide de transport étant comprise entre 200° et 600°C. Les grains de poivre P sont initialement contenus dans une trémie 43 et sont introduits dans le tuyau 40 par une écluse 44 qui consiste classiquement dans un conduit 45 muni de deux vannes 46a et 46b, l'introduction s'effectuant en ouvrant et en fermant alternativement lesdites vannes 46a et 46b. Le cyclone 42 comprend une première sortie 47 du fluide de transport chaud et une seconde sortie 48 des grains de poivre P débouchant sur un conduit 49, de très petite longueur, qui est relié à un second tuyau de transport pneumatique 50. Ce dernier présente des parois avantageusement calorifugées et comprend à une première extrémité des moyens de ventilation 41' et des moyens de refroidissement 12 et à son extrémité opposée un second cyclone 51, les grains de poivre P étant introduits dans le second tuyau de transport 50 à proximité des moyens de ventilation 41' et de refroidissement 12 et la température du fluide de transport étant comprise entre -200° et -20°C. Par ailleurs, le second cyclone 51 comprend une première sortie 52 du fluide de transport froid et une seconde sortie 53 des grains de poivre P débouchant sur des moyens de récupération tel qu'un récipient 54 par exemple. Ainsi, après leur introduction dans le premier tuyau de transport pneumatique 40, les grains de poivre P sont transportés par un fluide chaud, de l'air par exemple, jusqu'au cyclone 42 où les grains de poivre P sont séparés de l'air chaud pour être amenés par la conduite 49 jusqu'au second tuyau de transport pneumatique 50. Les grains de poivre P sont alors transportés par de l'air froid jusqu'au cyclone 51 séparant l'air froid des grains de poivre P décontaminés qui sont récupérés dans le récipient 54.

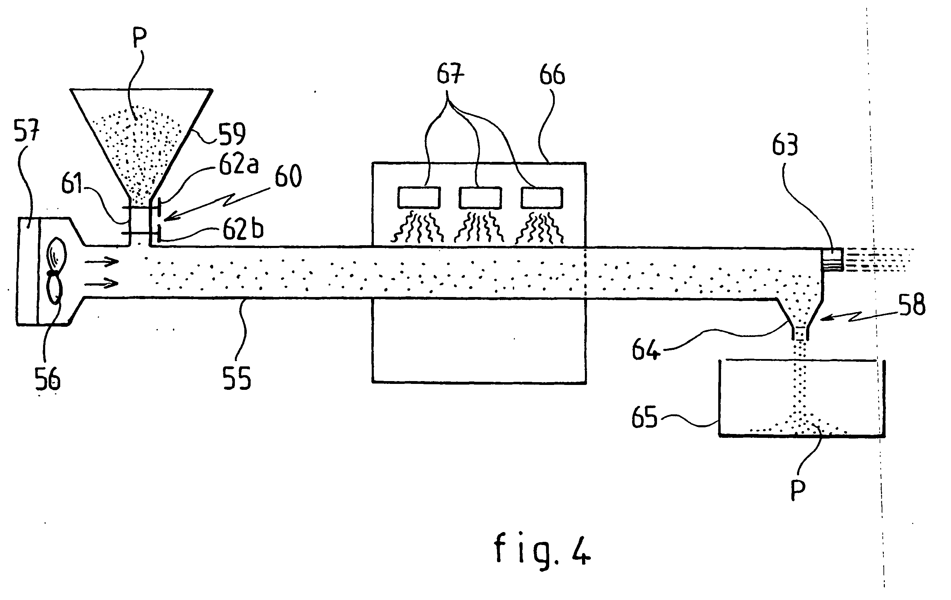

Enfin, selon une dernière variante d'exécution du dispositif en référence à la figure 4, le dispositif est constitué d'un tuyau de transport pneumatique 55 dont les parois sont avantageusement calorifugées comprenant à une première extrémité des moyens de ventilation 56 et des moyens de refroidissement 57 tels que du CO₂ liquide détendu à la pression atmosphérique par exemple et à son extrémité opposée un cyclone 58 permettant de séparer les grains de poivre P du fluide transport froid. On observera que la température du CO₂ liquide détendu à la pression atmosphérique et qui constitue le fluide de transport est de l'ordre de -80°C. Les grains de poivre P sont initialement contenus dans une trémie 59 et sont introduits dans le tuyau 55 par une écluse 60 qui consiste classiquement dans un conduit 61 muni de deux vannes 62a et 62b, l'introduction des graines de poivre P dans le tuyau 55 s'effectuant en ouvrant et en fermant alternativement lesdites vannes 62a et 62b. Le cyclone 58 comprend une première sortie 63 du fluide de transport froid et une seconde sortie 64 des grains de poivre P décontaminés qui sont récupérés dans un récipient 65. Le tuyau de transport 55 traverse, par ailleurs, une enceinte 66 dans laquelle sont positionnés des générateurs de micro-ondes 67 irradiants les grains de poivre P qui traversent l'enceinte 66 afin de les chauffer jusqu'à une température supérieure ou égale à 200°C. A la sortie de l'enceinte 66, les grains de poivre P n'étant plus irradiés, ces derniers sont refroidis par le fluide de transport froid à une température d'environ -80°C de sorte que les grains de poivre P récupérés dans le récipient 65 sont décontaminés.

Il est bien évident que les générateurs de micro-ondes 67 peuvent être substitués par des générateurs de rayonnements infrarouges, ultraviolets ou analogues sans sortir du cadre de l'invention. De plus, le dispositif peut ne comprendre qu'un unique générateur de ces rayonnements.

On exposera maintenant les étapes successives du procédé de décontamination microbiologique de produit pulvérulent à partir du dispositif le mettant en oeuvre représenté sur la figure 1, pris à titre d'exemple.

Dans cet exemple particulier, des grains de poivre entiers à traiter présentent sur leur surface une flore totale de 2,16 X 10⁶ micro-organismes par gramme de poivre dont des levures, des moisissures et des entérobactéries en quantité de 4,8 x 10³ par gramme de poivre. On entend par flore totale l'ensemble des micro-organismes vivant sur la surface desdits grains de poivre. Par ailleurs, la teneur en huiles essentielles qui sont les vecteurs des caractéristiques organoleptiques des grains de poivre est de l'ordre de 5% de leur poids.

En référence à la figure 1, la vanne 4 de la première enceinte 1 étant fermée, et la vanne 7 de l'entrée 5 étant ouverte, les grains de poivre P à traiter sont introduits dans ladite enceinte 1, puis la vanne 7 de l'entrée 5 est fermée de telle sorte que l'enceinte 1 soit hermétiquement close. Les moyens de chauffage 11 sont alors actionnés appliquant ainsi un flux de gaz chaud à une température de 300°C pendant environ 10 secondes sur les grains de poivre P.

Il va de soi que la température peut être supérieure à 300°C, la durée du traitement thermique étant alors de préférence inférieure à 10 secondes afin d'éviter la carbonisation des grains de poivre.

Immédiatement après ce premier traitement thermique chaud et les moyens de chauffage 7 étant arrêtés, la vanne 4 séparant les deux enceintes 1 et 2 est ouverte et la vanne 8 de la seconde enceinte est fermée de telle sorte que les grains de poivre P à traiter soient introduits dans la seconde enceinte 2 par tout moyen connu. La vanne 4 est alors fermée de manière que la seconde enceinte 2 soit hermétiquement close, puis les moyens de refroidissement 12 sont actionnés appliquant ainsi un flux de gaz froid à une température d'environ -80°C pendant environ 10 secondes sur les grains de poivre P. Après ce second traitement thermique froid, la vanne 8 de la sortie 6 est ouverte et les grains de poivre P sont évacués par le second conduit de transport pneumatique 10 jusqu'à ce que l'enceinte 2 soit vide. La vanne 8 est alors refermée et la vanne 7 de l'entrée 2 ouverte pour permettre l'introduction de nouveaux grains de poivre P.

Les grains de poivre, à l'issue de ce procédé, présentent une flore totale de l'ordre de 5 X 10³ micro-organismes par gramme de poivre et des levures, des moisissures et entérobactéries dans une quantité inférieure à 50 par gramme. De plus, leur teneur en huiles essentielles, à l'issue de cette opération, est de l'ordre de 4% de leur poids. Ainsi, il apparaît que le procédé de décontamination microbiologique procure, une décontamination, c'est-à-dire une réduction de l'ordre de 10³ fois des micro-organismes, notamment des levures et des moisissures, initialement présentes sur les grains de poivre, tout en conservant la teneur en huiles essentielles, c'est-à-dire tout en conservant les qualités organoleptiques des grains de poivre. De plus, l'apparence visuelle de ces grains de poivre ainsi traités reste inchangée.

On notera par ailleurs, que, de manière surprenante, le volume des grains de poivre a augmenté à l'issue du procédé, facilitant par la suite leur broyage. Les grains de poivre n'ayant pas cuits, c'est-à-dire que leur structure moléculaire étant restée globalement inchangée au cours du procédé, cette augmentation du volume des grains peut éventuellement s'expliquer par l'évaporation de l'eau résiduelle contenue dans ces grains. A cet effet, les grains de poivre peuvent avantageusement être humidifiés avant l'application des traitements thermiques afin de palier à leur déshydratation.

Par ailleurs, on a constaté que l'application d'un traitement froid à environ -40°C pendant une à dix secondes, permet de réduire d'environ 10² fois la flore totale, c'est-à-dire les levures et les moisissures des grains de poivre, sans réduire leur teneur en huiles essentielles de sorte que le procédé peut avantageusement comprendre un traitement thermique préalable à une température inférieure à 0°C pendant une courte durée, inférieure à 1 minute.

Il va de soi que les températures et les durées des traitements thermiques dépendent du type de produit pulvérulent à traiter et de leur taux de contamination.

Par ailleurs, il va de soi que le premier traitement thermique peut consister à faire transiter le produit pulvérulent dans une enceinte contenant un gaz chaud et/ou que le second traitement thermique peut consister à faire transiter ledit produit dans une enceinte contenant un gaz froid.

De plus, il est bien évident que le procédé peut consister dans une série de traitements alternés chaud et froid.

Enfin, le procédé de décontamination microbiologique peut naturellement s'appliquer à tous les types de produits pulvérulents tels que des poudres, des épices séchées et broyées, des graines ou analogues, et il est clair que les exemples que l'on vient de citer ne sont que des exemples particuliers en aucun cas limitatifs quant au domaine d'application de l'invention.

## Revendications

1. - Procédé de décontamination microbiologique de produits pulvérulents, de graines ou analogues **caractérisé en ce qu'**il consiste au moins à soumettre le produit pulvérulent à un premier traitement thermique consistant à appliquer un flux de gaz chaud sur le produit pulvérulent à une température comprise entre 200 °C et 600 °C pendant une durée inférieure à 30 secondes, immédiatement après à soumettre ledit produit à un second traitement thermique consistant à appliquer un flux de gaz froid sur le produit pulvérulent à une température inférieure à - 80°C pendant une durée inférieure à 30 secondes afin de détruire en tout ou partie les levures, les moisissures, les bactéries ou analogues, initialement présentes sur le produit pulvérulent tout en conservant ses qualités organoleptiques.

2. - Procédé de décontamination suivant la revendication 1 **caractérisé en ce que** le gaz est maintenu à une température constante.

3. - Procédé de décontamination suivant l'une quelconque des revendications précédentes **caractérisé en ce qu'**il comprend un traitement thermique préalable à une température inférieure à 0°C pendant une durée inférieure à une minute.

4. - Dispositif de décontamination microbiologique de produits pulvérulents, de graines ou analogues mettant en oeuvre le procédé suivant l'une quelconque des revendications précédentes, **caractérisé en ce qu'**il est constitué d'un premier tuyau de transport pneumatique (40) relié à une trémie (43) via une écluse (44) qui consiste dans un conduit (45) muni de deux vannes (46a, 46b), ledit premier tuyau de transport pneumatique (40) comprenant à une première extrémité des moyens de ventilation (41) et des moyens de chauffage (11) et à son extrémité opposée un premier cyclone (42) comprenant une première sortie (47) du fluide de transport et une seconde sortie (48) des produits pulvérulents, ladite seconde sortie (48) débouchant sur un conduit (49), ledit conduit (49) étant relié à un second tuyau de transport pneumatique (50), comprenant à une première extrémité des moyens de ventilation (41') et des moyens de refroidissement (12) à proximité dudit conduit (49) et à son extrémité opposée un second cyclone (51) comprenant une première sortie (52) du fluide de transport et une seconde sortie (53) des produits pulvérulents, ladite seconde sortie (53) débouchant sur des moyens de récupération (54).

5. - Dispositif de décontamination suivant la revendication 4 **caractérisé en ce** les moyens de chauffage (11) sont constitués de moyens de ventilation et d'une thermo-résistance.

6. - Dispositif de décontamination suivant la revendication 4 **caractérisé en ce que** les parois des tuyaux de transport pneumatique (40,50) sont calorifugées.

7. - Dispositif de décontamination suivant la revendication 4 **caractérisé en ce qu'**il comprend une trémie (43) pour l'introduction des produits pulvérulents dans le premier tuyau de transport pneumatique (40).

8. - Application du procédé de décontamination selon l'une quelconque des revendications 1 à 3 à des grains de poivre entiers.

## Claims

1. - Method of microbiological decontamination of powdery products, grains or the like **characterised in that** it consists in at least submitting the powdery product to a first heat treatment consisting in applying a flow of hot gas to the powdery product at a temperature between 200°C and 600°C for a duration less than 30 seconds, immediately after submitting said product to a second heat treatment consisting in applying a flow of cold gas to the powdery product at a temperature less than -80°C for a duration of less than 30 seconds in order to partially or totally destroy the yeasts, mould, bacteria or the like, initially present on the powdery product, while still conserving its organoleptic properties.

2. - Method of decontamination according to claim 1 **characterised in that** the gas is maintained at a constant temperature.

3. - Method of decontamination as claimed in any of the previous claims **characterised in that** it includes a prior heat treatment at a temperature less than 0°C for a duration of less than one minute.

4. - Device for microbiological decontamination of powdery products, grains or the like implementing the method according to any of the previous claims, **characterised in that** it is comprised of a first pneumatic transport pipe (40) connected to a bin (43) via a sluice-gate (44) which consists of a duct (45) equipped with two valves (46a, 46b), said first pneumatic transport pipe (40) including at a first end ventilation means (41) and heating means (11) and at its opposite end a first cyclone (42) including a first output (47) of transport fluid and a second output (48) of powdery products, said second output (48) emerging onto a conduct (49), said conduct (49) being linked to a second pneumatic transport pipe (50), including at a first end ventilation means (41') and cooling means (12) in proximity to said conduct (49) and at its opposite end a second cyclone (51) including a first output (52) of transport fluid and a second output (53) of powdery products, said second output (53) opening onto recovery means (54).

5. - Decontamination device according to claim 4 **characterised in that** heating means (11) are comprised of ventilation means and a thermo-resistance.

6. - Decontamination device according to claim 4 **characterised in that** the walls of the pneumatic transport pipes (40, 50) are thermally insulated.

7. - Decontamination device according to claim 4 **characterised in that** it includes a bin (43) for the introduction of powdery products in a first pneumatic transport pipe (40).

8. - Application of the method of decontamination as claimed in any of claims 1 to 3 to whole pepper grains.

## Patentansprüche

1. - Verfahren zur mikrobiologischen Dekontamination von pulverförmigen Produkten, Körnern oder ähnlichem, **dadurch gekennzeichnet, dass** es darin besteht, dass das pulverförmige Produkt mindestens einer ersten Wärmebehandlung unterzogen wird, die darin besteht, dass ein Strom warmen Gases bei einer Temperatur zwischen 200 °C und 600 °C über einen Zeitraum von weniger als 30 Sekunden auf das pulverförmige Produkt angewendet wird, das Produkt sofort danach einer zweiten Wärmebehandlung unterzogen wird, die darin besteht, dass ein Strom kalten Gases bei einer Temperatur von weniger als -80 °C über einen Zeitraum von weniger als 30 Sekunden auf das pulverförmige Produkt angewendet wird, um Hefen, Schimmelpilze, Bakterien oder ähnliches vollständig oder zum Teil zu zerstören, die anfänglich in dem pulverförmigen Produkt vorliegen, wobei gleichzeitig dessen organoleptischen Qualitäten beibehalten werden.

2. - Dekontaminationsverfahren nach Anspruch 1, **dadurch gekennzeichnet, dass** das Gas bei einer konstanten Temperatur gehalten wird.

3. - Dekontaminationsverfahren nach einem der vorhergehenden Ansprüche, **dadurch gekennzeichnet, dass** es eine vorherige Wärmebehandlung bei einer Temperatur von weniger als 0 °C über einen Zeitraum von weniger als einer Minute umfasst.

4. - Vorrichtung zur mikrobiologischen Dekontamination von pulverförmigen Produkten, Körnern oder ähnlichem, die das Verfahren nach einem der vorhergehenden Ansprüche umsetzt, **dadurch gekennzeichnet, dass** sie aus einem ersten pneumatischen Förderrohr (40) besteht, das über eine Schleuse (44), die aus einer mit zwei Schiebern (46a, 46b) versehenen Leitung (45) besteht, mit einem Trichter (43) verbunden ist, wobei das erste pneumatische Förderrohr (40) an einem ersten Ende Lüftungsmittel (41) und Heizmittel (11) umfasst und an seinem einem ersten Zyklon (42) gegenüberliegendem Ende einen ersten Ablass (47) für Förderfluid und einen zweiten Ablass (48) für pulverförmige Produkte umfasst, wobei der zweite Ablass (48) in einer Leitung (49) mündet, wobei die Leitung (49) mit einem zweiten pneumatischen Förderrohr (50) verbunden ist, das an einem ersten Ende Lüftungsmittel (41') und Kühlmittel (12) in der Nähe der Leitung (49) umfasst und an seinem einem zweiten Zyklon (51) gegenüberliegendem Ende einen ersten Ablass (52) für Förderfluid und einen zweiten Ablass (53) für pulverförmige Produkte umfasst, wobei der zweite Ablass (53) in den Rückgewinnungsmitteln (54) mündet.

5. - Dekontaminationsvorrichtung nach Anspruch 4, **dadurch gekennzeichnet, dass** die Heizmittel (11) aus Lüftungsmitteln und einem Thermoelement bestehen.

6. - Dekontaminationsvorrichtung nach Anspruch 4, **dadurch gekennzeichnet, dass** die Wände der pneumatischen Förderrohre (40, 50) wärmegedämmt sind.

7. - Dekontaminationsvorrichtung nach Anspruch 4, **dadurch gekennzeichnet, dass** sie einen Trichter (43) zur Einführung von pulverförmigen Produkten in das erste pneumatische Förderrohr (40) umfasst.

8. - Anwendung des Dekontaminationsverfahrens nach einem der Ansprüche 1 bis 3 auf ganze Pfefferkörner.
